# EUROPEAN PATENT APPLICATION

(11) **EP 3 508 199 A1**
(43) Date of publication of application: **10.07.2019**
(21) Application number: 18150490.3
(22) Date of filing: 05.01.2018
(51) Int. Cl.: A61K 9/20, A61K 31/192, A61K 31/205

(54) **IMMEDIATE-RELEASE PHARMACEUTICAL COMPOSITIONS CONTAINING KETOPROFEN LYSINE SALT**

(71) Applicant: Dompé farmaceutici S.p.A., 20122 Milan (IT)
(72) Inventor: CANTARINI, Marco, 22100 Como (CO) (IT); GENTILE, Marco Maria, 67100 L'Aquila (AQ) (IT); PROTA, Lucia, 84043 Agropoli (SA) (IT)
(74) Representative: Viganò, Elena

(57) **Abstract**

The present invention in general relates to an immediate-release pharmaceutical composition containing ketoprofen lysine salt and mannitol. In particular, the present invention relates to an immediate-release pharmaceutical composition in the form of tablet. The invention also provides a process for manufacturing such composition.

## Description

The present invention in general relates to an immediate-release pharmaceutical composition containing ketoprofen lysine salt and mannitol. In particular, the present invention relates to an immediate-release pharmaceutical composition in the form of tablet. The invention also provides a process for manufacturing such composition.

### BACKGROUND OF THE INVENTION

Ketoprofen is one of the propionic acid class of nonsteroidal anti-inflammatory drugs (NSAIDs) with analgesic and antipyretic effects.

Ketoprofen is generally prescribed for arthritis-related inflammatory pains or severe toothaches that result in the inflammation of the gums. Ketoprofen topical patches are being used for treatment of musculoskeletal pain.

Ketoprofen can also be used for treatment of some pain, especially nerve pain such as sciatica, postherpetic neuralgia and referred pain for radiculopathy, in the form of a cream, ointment, liquid, spray, or gel, which may also contain other agents. Ketoprofen lysine salt (KLS) has been assessed to exert the same anti-inflammatory, analgesic and antipyretic activities than the parent drug, ketoprofen.

The salification of ketoprofen with the amino-acid lysine has been shown to remarkably increase the solubility profile of ketoprofen in water, allowing the development of liquid and solid oral dosage forms.

From literature and in accordance with Biopharmaceutical Classification System (BCS) ketoprofen is assigned to BCS Class II due to its high permeability and solubility profile while low solubility of the highest dose at pH:1.2. Notwithstanding this assessment if we consider, for example, the dose of 40 mg of ketoprofen lysine salt (i.e. corresponding to 25 mg ketoprofen) we can see that the dose is completely soluble also at pH:1.2.

The solubility of ketoprofen lysine salt at pH:1.2 at 37°C is 0.29 mg/mL corresponding to a dose /solubility ratio of 138 ml (i.e. lower than 250 ml requested).

Hence on the base of literature and experimental data, for example a dose of 40 mg of ketoprofen lysine salt can be assigned to BCS Class I having high solubility and high permeability characteristics.

It is particularly desirable to provide a composition suitable for immediate release of the drug substance, which is stable and has a good dissolution profile and good general tolerability. In addition, it is the object of the present invention to provide a composition having good chemical and mechanical properties such as good uniformity, friability, hardness (resistant to crushing), and disintegration time.

### DEFINITIONS

Unless otherwise defined, all terms of art, notations and other scientific terminology used herein are intended to have the meanings commonly understood by those of skill in the art to which this disclosure pertains. In some cases, terms with commonly understood meanings are defined herein for clarity and/or for ready reference; thus, the inclusion of such definitions herein should not be construed to represent a substantial difference over what is generally understood in the art.

Within the framework of the present description and in the subsequent claims, except where otherwise indicated, all numbers expressing amounts, quantities, percentages, and so forth, are to be understood as being preceded in all instances by the term "about". Also, all ranges of numerical entities include all the possible combinations of the maximum and minimum numerical values and all the possible intermediate ranges therein, in addition to those specifically indicated hereafter.

The terms "comprising", "having", "including" and "containing" are to be construed open-ended terms (i.e. meaning "including, but not limited to") and are to be considered as providing support also for terms as "consist essentially of", "consisting essentially of", "consist of" or "consisting of".

The terms "consist essentially of", "consisting essentially of" are to be construed as semi-closed terms, meaning that no other ingredients which materially affects the basic and novel characteristics of the invention are included (optional excipients may thus included).

The terms "consists of", "consisting of" are to be construed as closed terms.

For the purposes of the present invention, with the term "immediate-release" is intended to indicate a composition which releases total amount of ketoprofen lysine salt from the composition in less than 1 hour, in a media ranging between a pH of 1 and a pH of 6.8.

The term "particle size distribution" (PSD) as used herein refers to the relative percentages by volume of each of the different size fractions of a particulate matter. The particle size distributions of the present application can be measured using laser light diffraction equipment, such as Malvern Mastersizer® 2000. Particle size is determined by measuring the angular distribution of laser light scattered by a homogeneous suspension of particles. The size distribution is determined from the light scattering data using the theory of light scattering developed by Gustav Mie. Other types of equipment are also suitable to determine particle size distribution. Laser light diffraction results can be expressed by d(0.9) and/or d(0.5) and/or d(0.1) median particle size values, which are based on a volume distribution.

The d(0.5) is the size in microns that splits the distribution with half above and half below this diameter. Thus, a d(0.9) comprised between 150 µm and 250 µm means that 90% by volume, of the particles, have a volume below a value in this range.

Likewise, a d(0.5) greater than 65 µm means that 50% by volume, of the particles, have a diameter greater than 65 µm; and a d(0.1) greater than 1.5 µm means that 10% by volume, of the particles, have a diameter greater than 1.5 µm.

### SUMMARY OF THE INVENTION

The present inventors, during the development of ketoprofen lysine salt immediate release tablet, surprisingly have found that higher disintegration rate (Pharmacopoeia) did not correlate with higher dissolution rate (Pharmacopoeia) at pH 1.2.

Generally, the disintegration time is the preferred test to screen immediate release tablet due to its rapid execution and good predictability of the release profile of drug substance. Dissolution test is normally performed subsequently on the tablets with best disintegration rate.

The present inventors have found that, even though the mannitol-based tablets presented higher values of disintegration time than CaHPO₄-based tablets, they unexpectedly showed a more rapid dissolution profile.

More particularly, according to a first aspect, the present invention relates to an immediate-release pharmaceutical composition containing ketoprofen lysine salt (KLS) having a particle size distribution with a d(0.9) comprised between 150 µm and 250 µm and/or a d(0.5) greater than 65 µm and/or a d(0.1) greater than 1.5 µm as the active principle, and mannitol wherein the ratio of ketoprofen lysine salt to mannitol is from about 100:100 to about 100:250.

Advantageously, the immediate-release compositions of the present invention are suitable for solid dosage forms, preferably tablets, for oral administration with water. The immediate-release pharmaceutical compositions of the present invention, preferably in the form of tablets, have a good hardness, friability, disintegration time and a good uniformity of the content as well as a good dissolution profile and a good general tolerability.

A second aspect of the present invention provides a process for preparing the pharmaceutical composition according to the first aspect by direct compression.

### DESCRIPTION OF THE FIGURES

**Figure 1** shows the dissolution profile of batches according to the invention containing different superdisintegrants at pH 1.0;
**Figure 2** shows dissolution profiles of batches according to the invention containing different fillers and superdisintegrants at pH 1.0;
**Figure 3** shows dissolution profiles of batches (reference) containing different fillers and superdisintegrants at pH 1.0;
**Figure 4** shows dissolution profiles of dyed and not dyed coated tablets at pH 1.0;
**Figure 5** shows the dissolution profile of batches according to the invention at pH 1.0;
**Figure 6** shows dissolution profiles of tablets produced with different compression forces, at pH 1.0;
**Figure 7** shows the particle size distribution of the not-micronized ketoprofen lysine salt;
**Figure 8** shows the particle size distribution of the micronized ketoprofen lysine salt;
**Figure 9** shows dissolution profiles of bacthes with ketoprofen lysine salts with different particle size distribution at pH 1.0.

### DETAILED DESCRIPTION OF THE INVENTION

As it will be disclosed in details in the Experimental Section, the present inventors have found that a pharmaceutical composition containing ketoprofen lysine salt having specific particle size distribution and mannitol allows to obtain immediate-release tablets suitable for oral administration with water.

Accordingly, a first object of the present invention is an immediate-release pharmaceutical composition containing ketoprofen lysine salt (KLS) having a particle size distribution with a d(0.9) comprised between 150 µm and 250 µm and/or a d(0.5) greater than 65 µm and/or a d(0.1) greater than 1.5 µm as the active principle, and mannitol wherein the ratio of ketoprofen lysine salt to mannitol is from about 100:100 to about 100:250.

Mannitol is used as binder for direct compression and as filler.

According to a preferred embodiment of the present invention, the mannitol has a particle size distribution with maximum of 35% greater than 150 µm and/or minimum of 70% greater than 75 µm.

The pharmaceutical composition according to the present invention, further comprising at least one superdisintegrant.

The superdisintegrants may be present in an amount ranging from 25 to 50%, preferably from 30 to 45% and more preferably from 35 and 40% by weight of the amount of ketoprofen lysine salt.

Suitable superdisintegrants for the present invention may be selected from the group comprising cross-linked polyvinyl pyrrolidone (crospovidone), cross-linked carboxymethylcellulose sodium (croscarmellose), sodium starch glycolate, pregelatinized starch and mixtures thereof. The preferred superdisintegrant is crospovidone.

In a preferred embodiment, the pharmaceutical composition according to the invention further comprising at least one lubricant and/or at least one glidant. Lubricant as described herein may be selected from a group comprising magnesium stearate, stearic acid, talc, sodium laurylsulfate, sodium stearyl fumarate, glyceryl behenate, and mixtures thereof. The preferred lubricants are sodium laurylsulfate and sodium stearyl fumarate.

A hydrophilic lubricant, like sodium stearyl fumarate, can be used in order to keep the disintegration time as short as possible, in order to guarantee a prompt release of the API. In particular, it gives faster dissolution rates, harder tablets, protection from over-blending, faster formulation development and scale-up and enhanced lubrication efficiency.

The lubricant may be present in an amount ranging from 0 to 4.0 %, preferably from 1.5 to 2.5 and more preferably from 1.75 to 2.25 % by weight of the composition. Glidant as described herein may be selected from the group comprising colloidal silica, talc, and mixtures thereof. The preferred glidant is colloidal silica.

The glidant may be present in an amount ranging from 0 to 2.0 %, preferably from 0.5 to 1.5 % and more preferably from 0.75 to 1.25 % by weight of the composition. The lubricants and glidants can be used in order to improve the dissolution rate of the API in the first minutes, in order to have a rapid effect in terms of release of the API. The immediate-release pharmaceutical composition according to the present invention is in the form of tablet.

The present inventors have surprisingly found that the selection of a specific mix of binders and disintegration agents promotes the breakup of the tablet in an aqueous environment thereby increasing the available surface area and promoting a more rapid release of the drug substance.

An immediate-release tablet according to the present invention is obtainable by direct compression with a tableting strength from 7 to 15 kN, preferably from 7 to 10 kN. Other ingredients, such as a coating film can also be used for organoleptic compliance. Suitable film-coating systems for the present invention may be the Opadry HPMC-based or PVA-based, preferably PVA-based.

Opadry is a mixture of polyvinyl alcohol or hydroxypropylmethylcellulose, polyethylene glycol / macrogol, titanium dioxide, talc and pigments.

The presence of a dye does not influence the dissolution rate of the tablets.

The second object of the present invention is a process for the preparation of an immediate-release tablet according to the invention, comprising the following steps:
a) providing ketoprofen lysine salt particles having a particle size distribution with a d(0.9) comprised between 150 µm and 250 µm and/or a d(0.5) greater than 65 µm and/or a d(0.1) greater than 1.5 µm, and mannitol wherein the ratio of ketoprofen lysine salt to mannitol is from about 100:100 to about 100:250;
b) mixing the ingredients of step a) in a suitable mixer to achieve a homogeneous mixture;
c) optionally mixing the blend of step b) with at least one superdisintegrant, and/or at least one lubricant, and/or at least one glidant until a homogeneous powder is obtained;
d) compressing the powder mixture of step c) into a tablet;
e) optionally coating the tablet of step d).

### Phase b) and c) - Powder mixing

Each ingredient has to be sieved to remove possible agglomerates, using a mesh size of 1 mm.

API is mixed with part of the total amount of the glidant; then the superdisintegrant, one of the lubricants (such as SDS) and a part of the filler are added and mixed with the first fraction. This procedure will mimic a geometric dilution of the API, and will contribute to its homogeneous dispersion in the final mixture.

The last step consist of the addition of the other lubricant (such as sodium stearyl fumarate), the glidant and the rest of the filler.

### Phase d) - Tableting

Different rotational speeds and compression forces were tested in order to obtain a tablet with the desired technological properties (crushing strength, thickness, average mass, friability, disintegration time, dissolution rate of the API).

The evaluation of the ejection forces and the aspect of the tablets were relevant to choose the optimal conditions.

### Phase e) - Film-coating

In order to obtain a taste-masking film coating a low viscosity polymer was chosen, the amount of that was selected considering the visual aspect of the tablets in terms of color uniformity, appearance and the weight.

A blue color was chosen for a distinctive recognition of the product, and for compliance purposes. The presence of a dye does not influence the dissolution rate. In order to mask the API taste during the administration and to have a product recognizable and appealing, a non-functional film-coating system can be added. Suitable film-coating systems for the present invention may be the Opadry HPMC-based (Opadry II 57U18539) or PVA-based (Opadry II 85F18422), preferably PVA-based.

Opadry is a mixture of polyvinyl alcohol or hydroxypropylmethylcellulose, polyethylene glycol / macrogol, titanium dioxide, talc and pigments.

To assess the ability of the Opadry coating to cover the off-taste of the tablets, the uniform distribution of the dye was monitored during the deposition of the film. It was observed that, after the application of 6 mg/tablet of Opadry, the coloration resulted homogeneous and consistent from tablet to tablet. This was taken as a signal of the end of the coating process. The weight gain of the tablets suggests that an even layer of coating film was applied on the surface of the tablet, therefore giving a taste-masking effect to the tablet itself.

The final weight of the tablet (156 mg) and the small dimension (7 mm diameter) was chosen to increase compliance of patients allowing an easy swallowing of the product.

Possible immediate-release pharmaceutical compositions of the invention are provided in the following examples, which, however, are only intended to illustrate and not to limit the invention.

### EXAMPLES

### EXAMPLE 1

The formulations according to the present invention as described in the Table 1 were prepared and tested. The tablets were prepared as reported above, using a direct compression.

**Table 1**

| **Ingredients** | **05214 F** | **05914 F** | **06014 F** |
|---|---|---|---|
| **Mannitol (Pearlitol 100)** | 62,00 % | 62,00 % | 62,00 % |
| **KLS** | 26,67% | 26,67% | 26,67% |
| **Crospovidone** | **10,00%** | - | - |
| **Croscarmellose sodium** | - | **10,00%** | - |
| **Sodium starch glycolate** | - | - | **10,00%** |
| **Sodium stearylfumarate** | 1,33% | 1,33% | 1,33% |

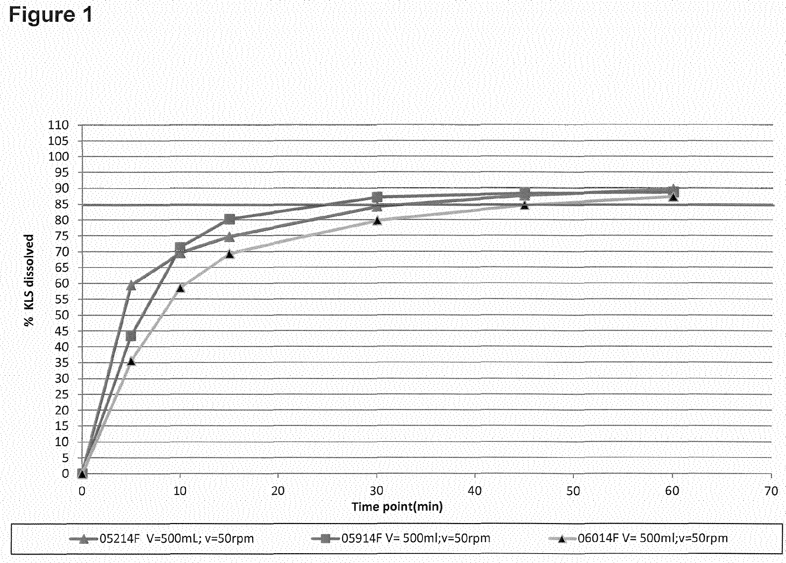

The dissolution profiles of the batches containing different superdisintegrants at pH 1.0 are reported in **Figure 1****.** The dissolution tests are carried out according to Ph. Eur. 2.9.3, current edition (37°C, 900 ml, 50 rpm) on 12 tablets sampling after 5, 10, 15, 30, 45 and 60 minutes. Since pH 1.0 corresponds to the profile which best resembles the stomach conditions (where the dissolution takes place) and where KSL results less soluble than pH 4.5 and 6.8, it could be considered as critical and for this reason it was selected as pH dissolution medium. In addition, the formulations according to the invention is intended to dissolve rapidly in the stomach and to have a prompt bioavailability.

As shown in the Figure 1, while croscarmellose gives the highest plateau concentration of KLS in time, crospovidone seems to be quicker in the first minutes of dissolution.

### EXAMPLE 2

The formulations according to the present invention as described in the Table 2 were prepared and tested. The tablets according to the invention were prepared as reported above, using a direct compression.

The formulations were prepared using these concentrations of superdisintegrants:
- Crospovidone (10.00%)
- Croscarmellose sodium (5.00%) + crospovidone (5.00%)
- Croscarmellose sodium (10.00%)

Two different diluents were tested in the same conditions, to see if the behavior of the superdisintegrant changes with the presence of calcium phosphate instead of mannitol.

**Table 2**

| | **05214 F** | **05814 F** | **05914 F** | **04814 F** | **05514 F** | **04014 F** |
|---|---|---|---|---|---|---|
| **Ingredients** | **DILUENT : MANNITOL** | | | **DILUENT : CALCIUM PHOSPHATE** | | |
| **Mannitol (Pearlitol 100)** | 62.00 % | 62.00 % | 62.00 % | - | - | - |
| **Ca₂HPO₄ (Di Cafos A150)** | - | - | - | 67.00% | 62.00% | 67.00% |
| **KLS** | 26.67% | 26.67% | 26.67% | 26.67% | 26.67% | 26.67% |
| **Crospovidone** | **10.00%** | **5.00%** | - | - | **5.00%** | **5.00%** |
| **Croscarmellose sodium** | - | **5.00%** | **10.00%** | **5.00%** | **5.00%** | - |
| **Sodium stearyl fumarate** | 1.33% | 1.33% | 1.33% | 1.33% | 1.33% | 1.33% |

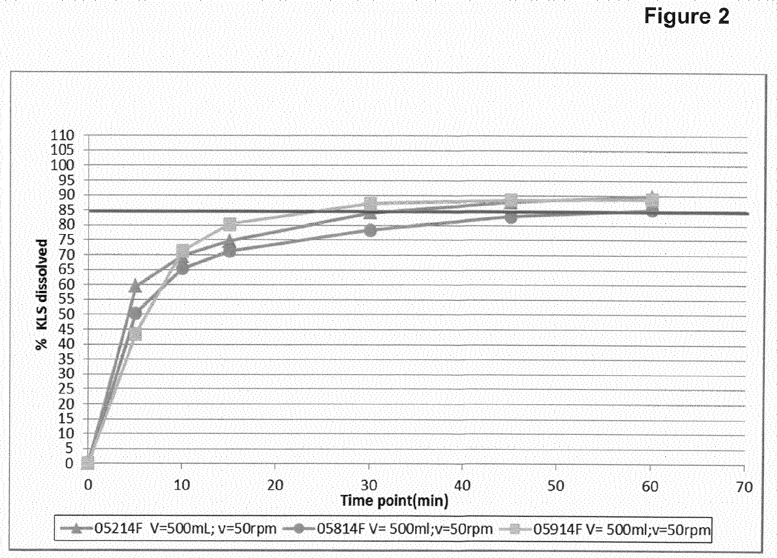

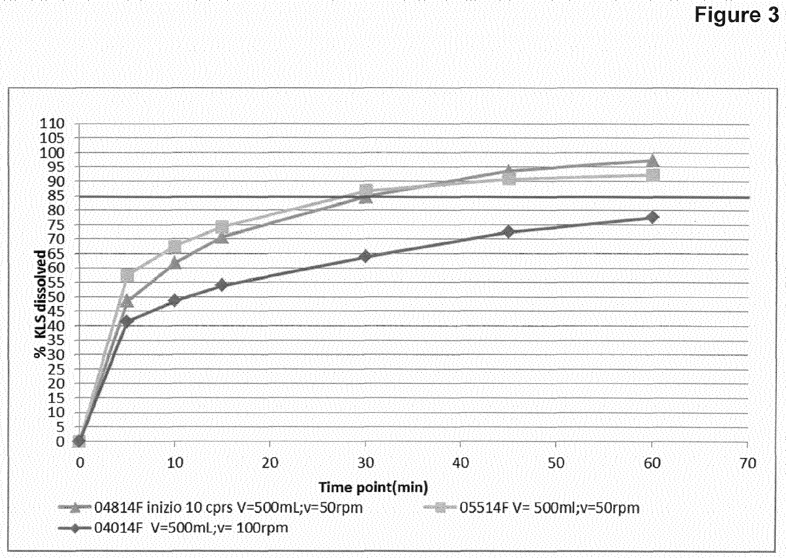

The dissolution profiles of batches containing different diluents and superdisintegrants at pH 1.0 are reported in **Figures 2** **and** **3****.**

The tablets prepared using mannitol as diluent according to the present invention (Figure 2), showed the quicker dissolution with respect to those prepared using calcium phosphate (Figure 3). The mannitol-based tablets dissolve rapidly in the stomach and have a prompt bioavailability.

### EXAMPLE 3

A formulation according to the present invention as described in the Table 3 was prepared and tested. The tablets according to the invention were prepared as reported above, using a direct compression and a final film coating.

**Table 3**

| **Ingredients** | **07814F mg** | **07814F %** |
|---|---|---|
| **KLS** | 40.00 | 25.64% |
| **Mannitol** | 93.00 | 59.62% |
| **Crospovidone** | 15.00 | 9.62% |
| **Sodium stearyl fumarate** | 2.00 | 1.28% |
| **Opadry II 85F205092 Blue** | 6.00 | 3.85% |
| **TOT** | 156.00 | 100% |

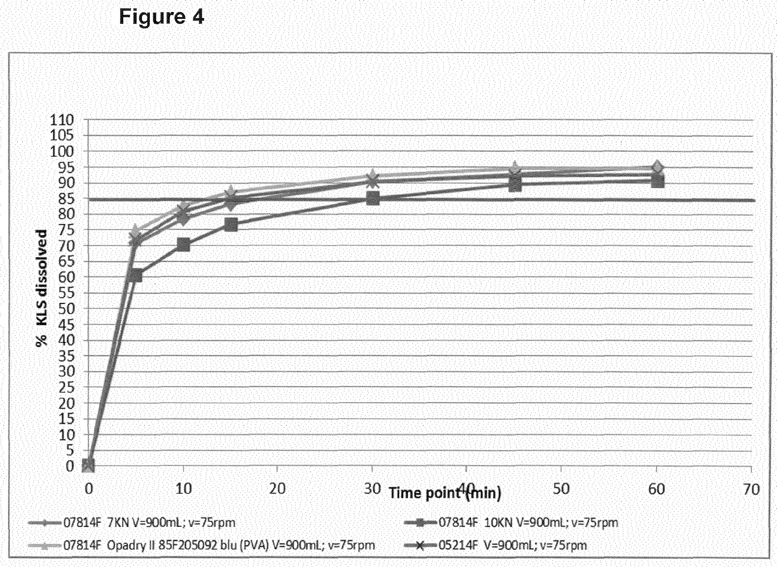

Results shown in **Figure 4** indicate that the presence of a dye does not influence the dissolution rate of the tablets.

### EXAMPLE 4

A composition containing also colloidal silica and sodium laurylsulfate according to the present invention as described in the Table 4 was prepared and tested.

**Table 4**

| **Ingredients** | **08114F** |
|---|---|
| **KLS** | 26,67% |
| **Mannitol** | 60,00% |
| **Crospovidone** | 10,00% |
| **SLS** | 1,00% |
| **Silica, Colloidal anhydrous** | 1,00% |
| **Sodium stearyl fumarate** | 1,33% |

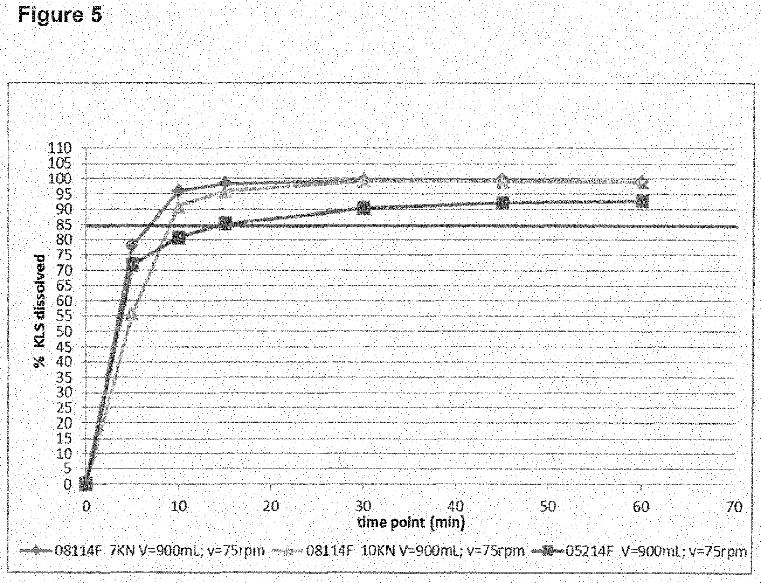

The results shown in **Figure 5** indicate that the composition reported in Table 4, pressed at 7KN, improves the dissolution profile of the API in the tablet at pH 1.0.

### EXAMPLE 5

A composition containing a fine particles-grade of sodium laurylsulfate (e.g. Kolliphor SLS fine) according to the present invention as described in the Table 5 was prepared and tested.

**Table 5**

| | **09314F** | |
|---|---|---|
| **Ingredients** | **Amount (mg)** | **Formula %** |
| KLS | 40 | 25.64 |
| Pearlitol 100SD (Mannitol DC) | 89 | 57.05 |
| Kollidon CL (Crospovidone) | 15 | 9.62 |
| Kolliphor SLS fine (Sodium Laurylsulfate) | 1.5 | 0.96 |
| Aerosil 200 (Silica, Colloidal anhydrous) | 1.5 | 0.96 |
| PRUV (Sodium stearyl fumarate) | 3 | 1.92 |
| Opadry II 85F205092 Blue | 6 | 3.85 |
| **Tot** | **156** | **100** |

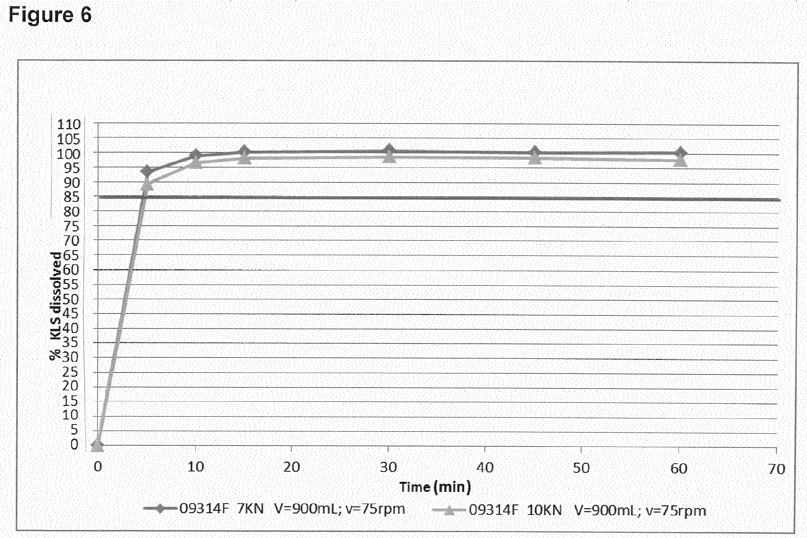

Different compression forces (7 KN and 10 KN) were used for the production of the tablets, using the same bulk. Dissolution profiles, shown in **Figure 6****,** demonstrate that both compression forces give good dissolution performances of the tablets.

### EXAMPLE 6

A formulation according to the present invention as described in the Table 5 was prepared and tested.

The dissolution profiles were evaluated at three different pH (pH 1.0, 4.5 and 6.8). The dissolution parameters are setting on the basis of the requirement reported in the guideline CPMP/EWP/QWP/1401/98 Rev.1. The results are reported in Tables 6-8.

**Table 6**

| Dissolution Rate Test (DRT) - *Dissolution medium pH 1.0* | | |
|---|---|---|
| **Time (minutes)** | **%KLS dissolved** | **Coefficient of variation (CV%)** |
| **0** | 0.00 | 0 |
| **5** | 91.33 | 2 |
| **10** | 99.19 | 1 |
| **15** | 100.00 | 1 |
| **30** | 100.21 | 2 |
| **45** | 100.02 | 2 |
| **60** | 99.36 | 2 |

**Table 7**

| Dissolution Rate Test (DRT) - *Dissolution medium pH 4.5* | | |
|---|---|---|
| **Time (minutes)** | **%KLS dissolved** | **CV%** |
| **0** | 0.00 | 0 |
| **5** | 93.17 | 3 |
| **10** | 97.72 | 3 |
| **15** | 98.33 | 2 |
| **30** | 97.59 | 2 |
| **45** | 96.61 | 2 |
| **60** | 95.69 | 1 |

**Table 8**

| Dissolution Rate Test (DRT) - dissolution medium pH 6.8 | | |
|---|---|---|
| **Time (minutes)** | **%KLS dissolved** | **CV%** |
| **0** | 0.00 | 0 |
| **5** | 95.82 | 3 |
| **10** | 97.47 | 2 |
| **15** | 101.84 | 2 |
| **30** | 101.88 | 2 |
| **45** | 100.86 | 2 |
| **60** | 99.67 | 2 |

As shown in the above dissolution profiles, the formulation according to the present invention ensures an overall complete release of the API at each pH tested.

### EXAMPLE 7

Two different ketoprofen lysine salts (micronized and not-micronized) were used and tested in their dissolution behavior.

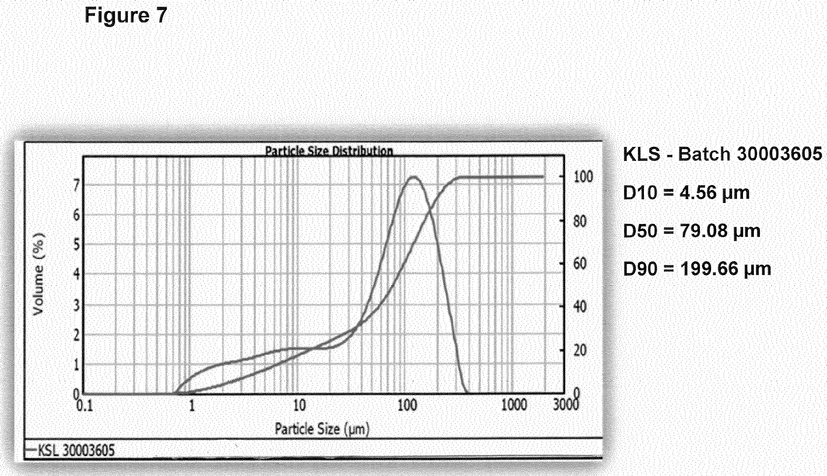

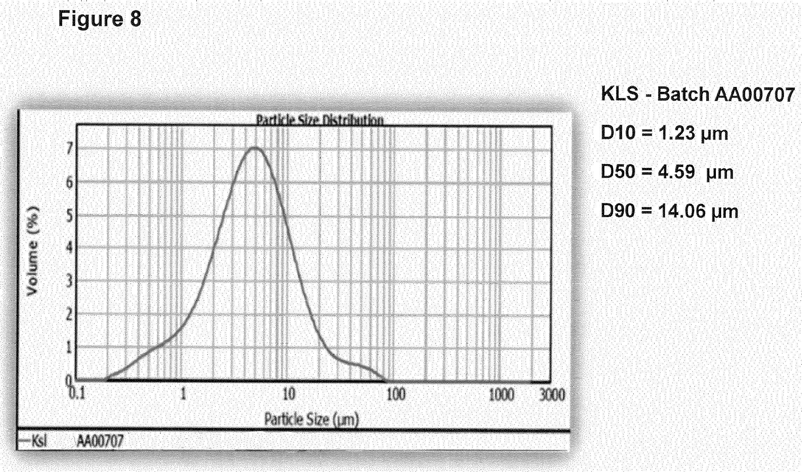

The KLS used were the not-micronized (batch 30003605), wherein its particle size distribution is shown in **Figure 7** and the micronized (batch AA00707), wherein its particle size distribution is shown in **Figure 8****.**

The formulations produced with the aforementioned KLSs are reported in Table 9.

**Table 9**

| | **05214 F** | **06714** F |
|---|---|---|
| **Mannitol (Pearlitol 100)** | 62.00 % | 60.00 % |
| **KLS - batch 30003605** | **26.67%** | - |
| **KSI - batch AA00707** | **-** | **33.67%** |
| **Crospovidone** | 10.00% | 5.00% |
| **Sodium stearyl fumarate** | 1.33% | 1.33% |

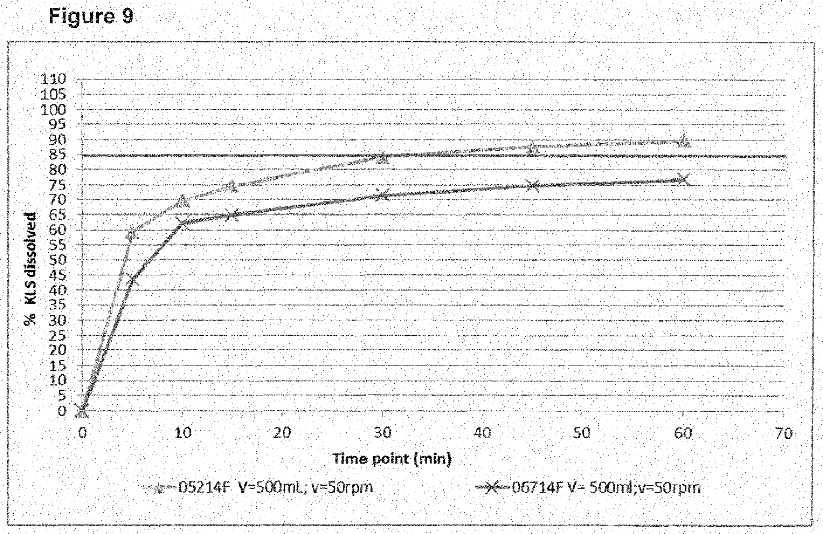

Dissolution profiles shown in **Figure 9** indicate that the use of a micronized KLS does not improve the dissolution rate of the tablets. Moreover, micronized KLS would result in a worse industrialization of the product, since it tends to stick to walls and gives poor flow properties to the bulk to be pressed.

Therefore, a particle size distribution according to the present invention allows to obtain immediate-release tablets dissolving rapidly in the stomach and having a prompt bioavailability.

## Claims

1. An immediate-release pharmaceutical composition containing ketoprofen lysine salt (KLS) having a particle size distribution with a d(0.9) comprised between 150 µm and 250 µm and/or a d(0.5) greater than 65 µm and/or a d(0.1) greater than 1.5 µm as the active principle, and mannitol wherein the ratio of ketoprofen lysine salt to mannitol is from about 100:100 to about 100:250.

2. The immediate-release pharmaceutical composition according to claim 1, wherein the mannitol has a particle size distribution with maximum of 35% greater than 150 µm and/or minimum of 70% greater than 75 µm.

3. The immediate-release pharmaceutical composition according to claim 1 or 2, further comprising at least one superdisintegrant.

4. The immediate-release pharmaceutical composition according to claim 3, wherein said at least one superdisintegrant is present in an amount ranging from 25 to 50% , preferably from 30 to 45% and more preferably from 35 to 40% by weight of the amount of ketoprofen lysine salt.

5. The immediate-release pharmaceutical composition according to claim 3 or 4, wherein said at least one superdisintegrant is selected from a group comprising crospovidone, croscarmellose, sodium starch glycolate, pregelatinized starch and mixtures thereof, preferably crospovidone.

6. The immediate-release pharmaceutical composition according to any one of the preceding claims, further comprising at least one lubricant and/or at least one glidant.

7. The immediate-release pharmaceutical composition according to claim 6, wherein said at least one lubricant is present in an amount ranging from 0 to 4.0%, preferably from 1.5 to 2.5% and more preferably from 1.75 to 2.25% by weight of the composition and/or said at least one glidant is present in an amount ranging from 0 to 2.0%, preferably from 0.5 to 1.5% and more preferably from 0.75 to 1.25% by weight of the composition.

8. The immediate-release pharmaceutical composition according to claim 6 or 7, wherein said at least one lubricant is selected from a group comprising magnesium stearate, stearic acid, talc, sodium laurylsulfate, sodium stearyl fumarate, glyceryl behenate and mixtures thereof, preferably sodium laurylsulfate and sodium stearyl fumarate.

9. The immediate-release pharmaceutical composition according to claim 6 or 7, wherein said at least one glidant is selected from a group comprising colloidal silica, talc, stearic acid and mixture thereof, preferably colloidal silica.

10. The immediate-release pharmaceutical composition according to any one of the preceding claims, in the form of tablet.

11. An immediate-release tablet according to claim 10, obtainable by direct compression with a tableting strength from 7 to 15 kN, preferably from 7 to 10 kN.

12. A process for the preparation of an immediate-release tablet according to claim 10 or 11, comprising the following steps:
a) providing ketoprofen lysine salt particles having a particle size distribution with a d(0.9) comprised between 150 µm and 250 µm and/or a d(0.5) greater than 65 µm and/or a d(0.1) greater than 1.5 µm, and mannitol wherein the ratio of ketoprofen lysine salt to mannitol is from about 100:100 to about 100:250;
b) mixing the ingredients of step a) in a suitable mixer to achieve a homogeneous mixture;
c) optionally mixing the blend of step b) with at least one superdisintegrant, and/or at least one lubricant, and/or at least one glidant until a homogeneous powder is obtained;
d) compressing the powder mixture of step c) into a tablet;
e) optionally coating the tablet of step d).
